# EUROPEAN PATENT SPECIFICATION

(11) **EP 3 097 915 B1**
(45) Date of publication and mention of the grant of the patent: **24.01.2024**
(21) Application number: 16178624.9
(22) Date of filing: 18.04.2012
(51) Int. Cl.: A61K 45/06, A61K 31/137, A61P 11/00, A61K 31/573, A61K 9/00, A61P 11/08

(54) **IMPROVED SUSPENSION FORMULATION OF A CORTICOSTEROID FOR ADMINISTRATION BY INHALATION**
VERBESSERTE SUSPENSIONSFORMULIERUNG EINES CORTICOSTEROIDS ZUR VERABREICHUNG MITTELS INHALATION
FORMULATION DE SUSPENSION AMÉLIORÉE D'UN CORTICOSTÉROÏDE POUR ADMINISTRATION PAR INHALATION

(30) Priority: 03.05.2011 EP 11164575
(43) Date of publication of application: 30.11.2016
(62) Divisional of application: 12719304.3
(73) Proprietor: Chiesi Farmaceutici S.p.A., 43122 Parma (IT)
(72) Inventor: CANTARELLI, Anna Maria, 43100 PARMA (IT); MINARI, Stefano, 43100 PARMA (IT); BASSI, Barbara, 43100 PARMA (IT)
(74) Representative: Script IP Limited

(56) References cited:
- WO-A1-03/086347
- WO-A1-2004/091576
- WO-A1-2009/074666
- WO-A2-00/25746
- WO-A2-2005/115332
- US-A1- 2004 208 831

## Description

The present invention relates to a formulation in form of aqueous suspension of drug particles of a corticosteroid to be administered by nebulisation, characterised by an optimal particle size distribution.

Also disclosed herein are processes for preparing said formulation, uses and kits thereof.

### BACKGROUND OF THE INVENTION

The method of delivering drugs by inhalation has been used for several years, and is the mainstay of the treatment of diseases that limit the respiratory flow, such as asthma and chronic bronchitis.

The advantages of inhalation over the systemic route include the fact that the drug is released directly at the site of action, thus preventing systemic side effects and resulting in a more rapid clinical response and a higher therapeutic index.

Among the various types of drug which are administered by inhalation for the treatment of the respiratory diseases, insoluble or poorly water soluble corticosteroids, such as beclomethasone dipropionate (BDP), mometasone furoate, flunisolide, budesonide, fluticasone propionate and others are of great importance. Said drugs could be administered by nebulisation as micronised particles in suspension in an aqueous phase that usually also contains surfactants and/or other excipients.

The efficacy of this form of administration depends on the deposit of a sufficient quantity of particles at the site of action.

In order to ensure an effective penetration into the lower respiratory tract of the patient, i.e. bronchioli and alveoli, one of the most important parameters is particle size, which must be of some micron.

Particles with a larger diameter are ineffective because they are deposited in the oropharyngeal cavity, and are therefore unable to reach the terminal branches of the respiratory tree; they can also give rise to local side effects, or may be absorbed through the buccal mucosa and give rise to systemic side effects.

It is important to ensure correct administration, and therefore therapeutic efficacy, that a homogenous dispersion of the particles in suspension is achieved, without the formation of aggregates. Said aggregates can indeed prejudice the efficacy of nebulisation as particles with a diameter exceeding 5-6 µm are unable to reach the preferential site of action. The formation of aggregates, especially "cakes", i.e. sets of highly compact suspended particles, can also give rise to problems of distribution and therefore of uniformity of dose during the filling of the formulation in the containers, making the administration less therapeutically effective, as the dose may be transferred incompletely from the vial to the bulb of the nebuliser apparatus by the patient.

During the manufacturing of the formulation, it would also be useful incrementing the wettability of the drug in such a way as to increase its speed of dispersion in the aqueous phase, a step that is often time-consuming.

Further, a mandatory requirement that should be met by pharmaceutical formulations for nebulisation is sterility, as confirmed by the various Acts and Directives governing their quality and safety.

The current trend is to produce inhalation formulations devoid of preservatives and bacteriostatics and including the fewest possible excipients, as it has been reported in the literature that some of the substances commonly used in said formulations might induce allergic reactions or give rise to irritation of the respiratory mucosae.

On the other hand, the limited choice of excipients could make the task of preparing physically stable formulations.

All these problems have been solved by the formulation of the present invention characterised by a selected very narrow and well-defined particle size distribution of the suspended particles.

In WO 00/25746, WO 03/086347, and WO 2004/054545, the applicant has disclosed processes for the preparation of aqueous suspensions of corticosteroids for nebulisation, but said documents are silent about a particle size distribution of the suspended particles fulfilling the characteristics outlined and claimed as follows.

### SUMMARY OF THE INVENTION

In a first aspect, the present invention is directed to a propellant-free sterile pharmaceutical formulation for administration for the prevention and/or treatment of a respiratory disease selected from asthma and chronic obstructive pulmonary disease by nebulisation to the lungs, said formulation comprising micronized particles of beclometasone dipropionate suspended in an aqueous solution at a concentration of 0.04% w/v, wherein i) no more than 10% of said suspended particles have a volume diameter [d(v,0.1)] lower than 0.7 micron, ii) no more than 50% of said particles have a volume diameter [d(v,0.5)] comprised between 1.6 micron and 1.8 micron;
and iii) at least 90% of said particles [d(v,0.9)] have a volume diameter lower than 4.0 micron, and wherein said particles have a particle size span, defined as [d(v,0.9)-d(v,0.1)]/d(v,0.5), comprised between 1.8 and 2.1 micron; wherein said volume diameters are measured by laser diffraction.

Also disclosed herein is a process for the preparation of the aforementioned formulation.

In another embodiment, the invention also relates to a vial filled with the claimed free formulation.

Also disclosed herein is the claimed formulation for use for the prevention and/or treatment of an inflammatory or obstructive airways disease such as asthma or chronic obstructive pulmonary disease (COPD).

Also disclosed herein is a method of preventing and/or treating an inflammatory or obstructive airways disease such as asthma or chronic obstructive pulmonary disease (COPD), which comprises administration by inhalation of an effective amount of the formulation of the invention.

Also disclosed herein is use of the claimed formulation in the manufacture of a medicament for the prevention and/or treatment of an inflammatory or obstructive airways disease such as asthma or chronic obstructive pulmonary disease (COPD).

In another embodiment, the invention refers to a kit comprising
a) the formulation of the invention filled in a unit-dose vial; and
b) a nebulizer.

### DEFINITIONS

The terms "active drug", "active ingredient", "active" and "active substance", "active compound" and "therapeutic agent" are used as synonymous.

The term "corticosteroids" refers to class of active ingredients having a hydrogenated cyclopentoperhydrophenanthrene ring system endowed with an antiinflammatory activity.

The term "propellant free" indicates that the formulation is not delivered in admixture with any of the commonly used aerosol propellants, such as hydrofluorocarbons, hydrocarbons, compressed gases, and the like.

The expression "insoluble or poorly water soluble" refers to an active ingredient having a solubility in water as defined in the European Pharmacopoeia Ed. 4th 2003, page 2891.

By the term "nebulisation", it is meant the generation of very fine liquid droplets for inhalation to the lungs by means of suitable devices called nebulisers.

By the term "sterile" it is meant a product which meets the criteria of sterility according to the European Pharmacopoeia (Ph. Eur. 1998, Chapters 2.6.1 and 5.1.1). Further regulations for sterility of the final product include the US Pharmacopoeia 23/NF 18, 1995, pp. 1686-1690 and 1963-1975.

In the present application, the particle size is quantified by measuring a characteristic equivalent sphere diameter, known as volume diameter by laser diffraction. The volume diameter (VD) is related to the mass diameter (MD) by the density of the particles (assuming a size independent density for the particles). Particle size distribution is described by: i) the volume median diameter (VMD) which corresponds to the diameter of 50 percent by volume of the particles [d(v,0.5)], and ii) the VD (MD) in micron of 10% and 90% of the particles [d(v,0.1) and d(v,0.9)].

Upon aerosolisation, the particle size is expressed as mass aerodynamic diameter (MAD) and the particle size distribution as mass median aerodynamic diameter (MMAD). The MAD indicates the capability of the particles of being transported suspended in an air stream. The MMAD corresponds to the mass aerodynamic diameter of 50 percent by weight of the particles.

In the context of the suspension formulations, the expression "physically stable" refers to formulations which exhibit substantially no growth in particle size or change in crystal morphology of the active ingredient over a prolonged period, are readily redispersible, and upon redispersion, do not flocculate so quickly as to prevent reproducing dosing of the active ingredient.

The expression "respirable fraction" refers to an index of the percentage of active particles which would reach the deep lungs in a patient.

The respirable fraction, also termed fine particle fraction, is evaluated using a suitable *in vitro* apparata such as Multistage Cascade Impactor or Multi Stage Liquid Impinger (MLSI) according to procedures reported in common Pharmacopoeias.

It is calculated by the ratio between the delivered dose and the fine particle mass (formerly fine particle dose).

The emitted dose is calculated from the amount of active ingredient collected on the filter; the delivered dose is calculated from the cumulative deposition in the apparatus, while the fine particle mass is calculated from the deposition on Stages 3 (S3) to filter (AF) corresponding to particles <6.4 microns. The term "prevention" means an approach for reducing the risk of onset of a disease.

The term "treatment" means an approach for obtaining beneficial or desired results, including clinical results. Beneficial or desired clinical results can include alleviation or amelioration of one or more symptoms or conditions, diminishment of extent of disease, stabilized (i. e. not worsening) state of disease, preventing spread of disease, delay or slowing of disease progression, amelioration or palliation of the disease state, and remission (whether partial or total), whether detectable or undetectable. The term can also mean prolonging survival as compared to expected survival if not receiving treatment.

### DETAILED DESCRIPTION OF THE INVENTION

The propellant-free sterile pharmaceutical formulation of the invention for administration through nebulisation comprises micronized crystalline particles of an insoluble or poorly-water soluble corticosteroid suspended in an aqueous phase.

The corticosteroid is beclometasone dipropionate.

The beclometasone dipropionate (BDP) could be in its anhydrous form or in its monohydrated form.

In a particular embodiment crystalline BDP is present as anhydrous form while in another particular embodiment, BDP is present as monohydrated form.

*Inter alia,* the two forms are commonly distinguished by their powder X-ray diffractometry pattern.

The suspended micronized particles of the drug are characterized by a selected, narrow, and well defined particle size distribution in such a way that: i) no more than 10% of said suspended particles have a volume diameter [d(v,0.1)] lower than 0.7 micron ii) no more than 50% of said particles have a volume diameter [d(v,0.5)] comprised between 1.6 and 1.8 micron; and iii) at least 90% of said particles [d(v,0.9)] have a volume diameter equal to or lower than 4.0 micron.

The suspended particles should also have a width of the particle size distribution expressed as a span comprised between 1.8 and 2.1.

According the Chew et al J Pharm Pharmceut Sci 2002, 5, 162-168, the span corresponds to [d(v,0.9)- d(v,0.1)]/d(v,0.5).

Advantageously, substantially all the suspended particles have a volume diameter comprised between 6 and 0.4 micron, preferably between 5.5 and 0.45 micron.

The particle size of the active substance is determined by measuring the characteristic equivalent sphere diameter, known as volume diameter, by laser diffraction as described above, preferably using a Malvern apparatus available from Malvern Instruments Ltd.

The formulation according to the invention exhibits a homogenous distribution of the particles as well as a higher level of physical stability in comparison to the formulations of the prior art, as the particles sediment more slowly and are less liable to form agglomerates.

Typically the aqueous phase comprises a pharmaceutically acceptable excipient selected from the group consisting of wetting agents, isotonicity agents, and optionally stabilisers and/or buffers for adjusting the pH.

The wetting agent may be selected from the group consisting of polysorbate 20, polysorbate 80, and sorbitan monolaurate. The isotonicity agent is typically sodium chloride.

If present, the stabilizer might be selected from complexing agents such as ethylenediaminetetraacetic acid (EDTA) or a salt thereof, i.e. the disodium salt, while the buffer might be a citric or a phosphate buffer.

Advantageously the pH of the formulation is comprised between 3.5 and 6.0.

Due to the particle size characteristics, and in particular due to the high surface area that is inversely proportional to the particle size distribution, the corticosteroid particles turn out to be more wettable and easy to be dispersed, and hence the formulation of the invention could be prepared using common procedures known in the art. For example, the preparation may be carried out in a turboemulsifier, an apparatus commonly used in the art.

Typically, the process of preparation comprises the following steps:
a) an aqueous solution comprising suitable the suitable excipients is prepared in a suitable tank, then transferred to a turboemulsifier provided with a turbine adapted for homogenising the suspension, and optionally with a mechanical agitator;
b) the active ingredient in the form of a micronised particles is added to the aqueous solution;
c) the suspended particles of the active ingredient are homogenised by stirring.

Advantageously, the preparation of the formulation may be performed using a turboemulsifier according to the conditions described in WO 00/25746, or preferably under vacuum according to the conditions described in WO 03/086374.

The formulation of the invention must be sterile. Said sterility requirement could be fulfilled in different ways.

For example, the formulation may be sterilised by subjecting the aqueous suspension obtained from a non-sterile micronized active ingredient to steam sterilization according to the conditions disclosed in EP 1599233.

Alternatively, the formulation can be prepared under aseptic conditions starting from micronised sterile particles of the corticosteroid.

In turn, the micronised sterile corticosteroid may be prepared by subjecting first the powder to micronisation, then to sterilization by irradiation or dry heating.

Preferably, the micronised BDP shall be sterilised by treatment with gamma rays under the conditions reported in WO 00/25746. Otherwise, the micronised corticosteroid may be sterilized by dry heating according to the conditions disclosed in EP 1032396, a process also known as tyndallization.

The micronised sterile corticosteroid may also be prepared by subjecting first the powder to sterilisation by means of irradiation with gamma or beta rays, and then to sterile micronisation, according to the teaching of WO 2007/062685.

The micronisation step is usually carried out by grinding using a conventional fluid energy mill such as the jet mill apparatus. Depending on the type of the apparatus and size of the batch, the person skilled in the art shall suitably adjust the milling parameters such as the operating pressure and the feeding rate to achieve the desired particle size.

Preferably the micronisation is carried out with the exclusion of moisture, more preferably using an inert gas such as nitrogen.

Advantageously, the micronized particles of the corticosteroid have a specific surface area comprised between 5.5 and 9.0 m²/g, preferably between 6.5 and 8.0 m²/g. The Specific Surface Area is determined by Brunauer-Emmett-Teller (BET) nitrogen adsorption method according to a procedure known in the art.

In a further embodiment, the micronised sterile particles of the corticosteroid could be obtained in the desired particle size by crystallization, followed by micronisation, both under aseptic conditions.

The concentration of active ingredient in the pharmaceutical formulations according to the invention depends on the utilized corticosteroid, the kind and severity of the disease, and the conditions (weight, sex, age) of the patient.

The concentration of BDP is 0.04% w/v.

The formulation of the invention could also comprise one or more additional active ingredients suspended or dissolved in the aqueous phase.

Further active ingredients that could advantageously be used are those useful for the treatment of respiratory diseases, for example short-acting or long-acting beta₂-agonists such as salbutamol (albuterol), fenoterol, salmeterol and formoterol or salts thereof or anticholinergics drugs such as ipratropium bromide.

In a preferred embodiment, the formulation may comprise salbutamol in form of sulfate salt dissolved in the aqueous phase.

The formulation of the invention may be distributed in suitable containers such as multidose vials or, preferably, unit-dose vials for single dosage administration. Said vials may be made of glass or plastic materials. For example plastic materials for preparing the unit-dose vials include low density polyethylene, high density polyethylene, polypropylene and polyesters.

The unit-dose vials may be pre-sterilised or, preferably, may be aseptically filled using the "blow, fill and seal" technology.

Advantageously, the unit-dose vials may have a volume of 1 ml, 2 ml, 2.5 ml, 3 ml, 4 ml or 5 ml, preferably of 2 ml.

As a consequence, this invention also relates a vial filled with the formulation of the invention.

The formulations of the invention are intended for administration by nebulisation using suitable apparatus known as nebulisers.

Any nebuliser can be used with the formulation of the invention.

Said nebulisers may produce the nebulised droplets by any method known to those skilled in the art, including compressed air (jet), ultrasonic waves, or vibration.

Typically the formulation is administered using jet nebulisers coupled with suitable compressors like for example LC PLUS^{®} or LC Sprint^{®} (Pari GmbH, Germany).

Therefore the invention is also directed to a kit comprising the pharmaceutical formulation provided herein filled in a unit-dose vial and a nebulizer.

Administration of the formulation of the invention may be indicated for the prevention and/or treatment of a wide range of conditions including respiratory disorders such as chronic obstructive pulmonary disease (COPD) and asthma of all types.

### EXAMPLES

### EXAMPLE 1: Sterile suspension formulation comprising micronised anhydrous BDP as active ingredient

The formulation according to the invention was prepared starting from a suitable micronised anhydrous BDP made sterile by gamma-irradiation at 3.17 KGy as described in WO 00/25746.

The formulation was distributed in unit-dose vials using the "blow, fill and seal" technology.

Its composition is reported in Table 1

**Table 1**

| Ingredients | Total quantity of preparation | Quantity per pharmaceutical unit |
|---|---|---|
| Sterile micronised BDP | 0.6 kg | (0.80 mg) |
| Polysorbate (Tween) 20 | 1.5 kg | (2.0 mg) |
| Sorbitan monolaurate | 0.3 kg | (0.40 mg) |
| Sodium chloride | 13.5 kg | (18.0 mg) |
| Water for injection q. s. for | 15001 | (2.0 ml) |

The nebulisation efficiency was evaluated using the LC Sprint^{®} nebulizer (Pari GmbH, Germany) in a Multi Stage Liquid Impinger (MLSI) according to procedures reported in the European Pharmacopoeia.

The respirable fraction of BDP turned out to be of about 24% with a MMAD of the particles of 7.5 micron (GSD¹⁾ ± 1.8).

¹⁾ GSD is the geometric standard deviation

### EXAMPLE 2: Particle-size analysis of preparations obtained according to example 1

The dimensional characteristics of the particles were evaluated by using a Malvern apparatus and by microscopy.

This type of test exploits the diffraction of a laser beam by the particles to determine the size distribution of the particles in suspension. The parameter considered is the volumetric diameter in µm of 10%, 50% and 90% of the particles, expressed as d(v,0.1), d(v,0.5) and d(v,0.9) respectively, which is determined by assuming that the particles have a geometrical shape equivalent to a sphere. The particle size span, defined as d(v,0.9) - d(v,0.1)]/d(v,0.5), is also calculated.

The results are reported in Table 2 for the purpose of comparison with those relating to a suspension obtained as described in WO 03/086347.

**Table 2**

| Particle size (µm, Malvern) | BDP (according to the invention) | BDP (according to WO 03/086347) |
|---|---|---|
| d(v,0.1) | 0.67 | 0.78 |
| d (v,0.5) | 1.62 | 2.97 |
| d (v,0.9) | 3.99 | 7.88 |
| d(v,0.9) - d(v,0.1]/d(v,0.5) | 2.05 | 2.39 |

As it can be appreciated the formulation according to the invention exhibits a narrower particle-size distribution.

### EXAMPLE 3: Sterile suspension formulation comprising micronised anhydrous BDP and salbutamol as active ingredients

The formulation was prepared as described in Example 1 and distributed in unit-dose vials using the "blow, fill and seal" technology.

Its composition is reported in Table 3

**Table 3**

| Ingredients | Total quantity of preparation | Quantity per pharmaceutical unit |
|---|---|---|
| Sterile micronised BDP | 0.6 kg | (0.80 mg) |
| Salbutamol sulphate | 1.446 kg | (1.928 mg) |
| Polysorbate (Tween) 20 | 1.5 kg | (2.0 mg) |
| Sorbitan monolaurate | 0.3 kg | (0.4 mg) |
| Sodium chloride | 13.5 kg | (18.0 mg) |
| Water for injection q. s. for | 1500 1 | (2.0 ml) |

The nebulisation efficiency was evaluated using the LC Sprint^{®} nebulizer (Pari GmbH, Germany) in a Multi Stage Liquid Impinger (MLSI) according to procedures reported in the European Pharmacopoeia.

The respirable fraction of BDP turned out to be of about 24%, while that of salbutamol turned out be of about 36%.

The MMAD of BDP particles is of 6.7 micron (GSD ± 2.0) and the MMAD of salbutamol particles is 5.5 micron (GSD ± 2.2).

## Claims

1. A propellant-free sterile pharmaceutical formulation for the prevention and/or treatment of a respiratory disease selected from asthma and chronic obstructive pulmonary disease by nebulisation to the lungs, said formulation comprising micronized particles of beclometasone dipropionate suspended in an aqueous solution at a concentration of 0.04% w/v, wherein i) no more than 10% of said suspended particles have a volume diameter [d(v,0.1)] lower than 0.7 micron, ii) no more than 50% of said particles have a volume diameter [d(v,0.5)] comprised between 1.6 micron and 1.8 micron; and iii) at least 90% of said particles [d(v,0.9)] have a volume diameter equal to or lower than 4.0 micron, and wherein said particles have a particle size span, defined as [d(v,0.9) - d(v,0.1)]/d(v,0.5), comprised between 1.8 and 2.1; wherein said volume diameters are measured by laser diffraction.

2. The formulation according to claim 1, wherein the specific surface area of the micronized particles of beclometasone dipropionate is comprised between 5.5 and 9.0 m²/g.

3. The formulation according to claim 2, wherein the specific surface area is comprised between 6.5 and 8.0 m²/g.

4. The formulation according to any of the preceding claims, wherein the aqueous solution comprises one or more pharmaceutically acceptable excipients selected from the group consisting of wetting agent, an isotonicity agent, and optionally a stabiliser and/or a buffer for adjusting the pH.

5. The formulation according to claim 4, wherein the wetting agent is selected from the group consisting of polysorbate 20, polysorbate 80, and sorbitan monolaurate.

6. The formulation according to claim 4 or 5, wherein the isotonicity agent is sodium chloride.

7. The formulation according to any one of the preceding claims further comprising one or more additional active ingredients suspended or dissolved in the aqueous phase.

8. The formulation according to claim 7, wherein the additional active ingredient is salbutamol sulfate.

9. A vial filled with a formulation according to any one of claims 1 to 8.

10. A kit comprising:
(a) a formulation according to any one of claims 1 to 8 filled in a unit-dose vial; and
(b) a nebulizer.

## Patentansprüche

1. Treibgasfreie, sterile pharmazeutische Formulierung zur Vorbeugung und/oder Behandlung einer Atemwegserkrankung, ausgewählt aus Asthma und chronisch obstruktiver Lungenerkrankung, durch Zerstäubung in die Lungen, wobei die genannte Formulierung mikronisierte Teilchen von Beclometasondipropionat umfasst, die in einer wässrigen Lösung in einer Konzentration von 0,04 Gew.-% suspendiert sind, wobei i) nicht mehr als 10% der genannten suspendierten Teilchen einen Volumendurchmesser [d(v,0,1)] kleiner als 0,7 Mikron aufweisen, ii) nicht mehr als 50% der genannten Teilchen einen Volumendurchmesser [d(v,0,5)] zwischen 1,6 Mikron und 1,8 Mikron aufweisen; und iii) mindestens 90% der genannten Teilchen [d(v,0,9)] einen Volumendurchmesser gleich oder kleiner als 4,0 Mikron aufweisen, und wobei die genannten Teilchen eine Teilchengrößenspanne, definiert als [d(v,0,9) - d(v,0,1)]/d(v,0,5), zwischen 1,8 und 2,1 aufweisen; wobei die genannten Volumendurchmesser durch Laserbeugung gemessen werden.

2. Formulierung nach Anspruch 1, wobei die spezifische Oberfläche der mikronisierten Teilchen von Beclometasondipropionat zwischen 5,5 und 9,0 m²/g liegt.

3. Formulierung nach Anspruch 2, wobei die spezifische Oberfläche zwischen 6,5 und 8,0 m²/g liegt.

4. Formulierung nach einem der vorhergehenden Ansprüche, wobei die wässrige Lösung einen oder mehrere pharmazeutisch verträgliche Hilfsstoffe enthält, die aus der Gruppe ausgewählt sind, die aus einem Benetzungsmittel, einem Isotonisierungsmittel und gegebenenfalls einem Stabilisator und/oder einem Puffer zur Einstellung des pH-Werts besteht.

5. Formulierung nach Anspruch 4, wobei das Benetzungsmittel ausgewählt ist aus der Gruppe bestehend aus Polysorbat 20, Polysorbat 80 und Sorbitanmonolaurat.

6. Formulierung nach Anspruch 4 oder 5, wobei das Isotonisierungsmittel Natriumchlorid ist.

7. Formulierung nach einem der vorhergehenden Ansprüche, die außerdem einen oder mehrere zusätzliche Wirkstoffe enthält, die in der wässrigen Phase suspendiert oder gelöst sind.

8. Formulierung nach Anspruch 7, wobei der zusätzliche Wirkstoff Salbutamol-Sulfat ist.

9. Fläschchen, das mit einer Formulierung nach einem der Ansprüche 1 bis 8 gefüllt ist.

10. Kit bestehend aus:
a) einer Formulierung nach einem der Ansprüche 1 bis 8, abgefüllt in ein Einzeldosis-Fläschchen; und
(b) einem Zerstäuber.

## Revendications

1. Formulation pharmaceutique stérile sans propulseur pour la prévention et/ou le traitement d'une maladie respiratoire choisie parmi l'asthme et la bronchopneumopathie obstructive chronique par nébulisation dans les poumons, ladite formulation comprenant des particules micronisées de dipropionate de béclométasone en suspension dans une solution aqueuse à une concentration de 0,04 % p/v, dans laquelle i) pas plus de 10 % desdites particules en suspension ont un diamètre volumique [d(v,0,1)] inférieur à 0,7 micron, ii) pas plus de 50 % desdites particules ont un diamètre volumique [d(v,0,5)] compris entre 1,6 micron et 1,8 micron ; et iii) au moins 90 % desdites particules [d(v,0,9)] ont un diamètre volumique inférieur ou égal à 4,0 microns, et dans laquelle lesdites particules ont une plage de taille de particules définie comme [d(v,0,9) - d(v,0,1)]/d(v,0,5), comprise entre 1,8 et 2,1 ; dans laquelle lesdits diamètres volumiques sont mesurés par diffraction laser.

2. Formulation selon la revendication 1, dans laquelle la surface spécifique des particules micronisées de dipropionate de béclométasone est comprise entre 5,5 et 9,0 m²/g.

3. Formulation selon la revendication 2, dans laquelle la surface spécifique est comprise entre 6,5 et 8,0 m²/g.

4. Formulation selon l'une quelconque des revendications précédentes, dans laquelle la solution aqueuse comprend un ou plusieurs excipients pharmaceutiquement acceptables choisis dans le groupe consistant en un agent mouillant, un agent d'isotonicité et éventuellement un stabilisant et/ou un tampon pour corriger le pH.

5. Formulation selon la revendication 4, dans laquelle l'agent mouillant est choisi dans le groupe consistant en du polysorbate 20, du polysorbate 80 et du monolaurate de sorbitane.

6. Formulation selon la revendication 4 ou 5, dans laquelle l'agent d'isotonicité est du chlorure de sodium.

7. Formulation selon l'une quelconque des revendications précédentes comprenant en outre un ou plusieurs principes actifs supplémentaires en suspension ou dissous dans la phase aqueuse.

8. Formulation selon la revendication 7, dans laquelle le principe actif supplémentaire est du sulfate de salbutamol.

9. Flacon rempli avec une formulation selon l'une quelconque des revendications 1 à 8.

10. Kit comprenant :
(a) une formulation selon l'une quelconque des revendications 1 à 8 introduite dans un flacon unidose ; et
(b) un nébuliseur.
